# EUROPEAN PATENT SPECIFICATION

(11) **EP 4 360 574 B1**
(45) Date of publication and mention of the grant of the patent: **05.11.2025**
(21) Application number: 23205159.9
(22) Date of filing: 23.10.2023
(51) Int. Cl.: A61B 18/12, A61B 18/14, A61B 18/00, A61B 34/20, A61B 90/00

(54) **POSITION TRACKING FOR PULSED FIELD ABLATION**
POSITIONSVERFOLGUNG FÜR GEPULSTE FELDABLATION
SUIVI DE POSITION POUR ABLATION PAR CHAMP PULSÉ

(30) Priority: 24.10.2022 US 202217971762
(43) Date of publication of application: 01.05.2024
(73) Proprietor: BIOSENSE WEBSTER (ISRAEL) LTD., 2066717 Yokneam (IL)
(72) Inventor: GOVARI, Assaf, 2066717 Yokneam (IL)
(74) Representative: Carpmaels & Ransford LLP

(56) References cited:
- WO-A1-2021/108312
- CN-A- 113 349 923
- US-A1- 2021 299 453

## Description

### FIELD OF THE DISCLOSURE

The present disclosure is related generally to the field of electrophysiology, and particularly to the administration of pulsed field ablation (PFA).

### BACKGROUND

In PFA, an electric field causes irreversible electroporation (IRE) of the cell membrane, thereby killing the cell. Typically, a PFA routine includes the application of a series of pulse trains, with an appropriate pause between each pair of successive pulse trains. CN113349923A describes an ablation system comprising an information processing module and a signal input and output module which are in communication connection. The signal input and output module is used for being connected with at least one medical catheter, and when the medical catheter outputs ablation energy, the signal input and output module obtains spatial position information of an ablation site according to the current position of the medical catheter and sends the spatial position information of the ablation site to the information processing module.

### BRIEF DESCRIPTION OF THE DRAWINGS

The present disclosure will be more fully understood from the following detailed description of examples thereof, taken together with the drawings, in which:
Fig. 1 is a schematic illustration of an example electrophysiology mapping and ablation system, in accordance with some examples of the present disclosure; and
Fig. 2 is a flow diagram for a method for applying PFA to tissue of a heart, in accordance with some examples of the present disclosure.

### DETAILED DESCRIPTION OF EXAMPLES

### OVERVIEW

In conventional PFA routines, a series of pulse trains is applied with a fixed amount of time (e.g., 0.5-10 s) between successive trains. If the ablation catheter moves significantly, relative to the tissue, before the entire series has been applied, the series is aborted. However, in some applications - e.g., when applying PFA within a heart of a subject - it may be difficult to avoid movement of the catheter relative to the tissue. As a result, it may be difficult to complete a PFA routine.

To address this challenge, examples of the present disclosure provide a modified PFA routine, which capitalizes on several observations:
(i) Each pulse train is typically short enough (e.g., 50-500 µs) such that any movement of the catheter during the pulse train is insignificant.
(ii) Movement of the catheter between pulse trains is often cyclical with a period of 0.5-3 s.
(iii) The pauses between successive pulse trains need not necessarily be of a fixed duration.

In view of these observations, examples of the present disclosure do not abort a PFA routine merely because the catheter moved, and do not require a fixed amount of time between successive pulses. Rather, the catheter is allowed to move cyclically between pulses. Provided the catheter returns to approximately the same location at which the most recent pulse train was applied, the next pulse train is applied.

In particular, following the application (or "delivery") of a first pulse train at a first location, the position of the catheter is tracked. If, following a predefined minimum duration (and, typically, prior to a predefined maximum duration) from the application of the first pulse train, the catheter is at a second location within a predefined distance from the first location, a second pulse train is applied. Otherwise, the series of pulse trains is aborted.

Similarly, following the application of the second pulse train, the position of the catheter is tracked. If, following the predefined minimum duration (and, typically, prior to the predefined maximum duration) from the application of the second pulse train, the catheter is within the predefined distance from the first location and/or the second location, a third pulse train is applied. Otherwise, the series of pulse trains is aborted.

Upon aborting a series of pulse trains, a warning to the user may be outputted. Alternatively or additionally, the new location of the catheter may be selected for ablation.

### SYSTEM DESCRIPTION

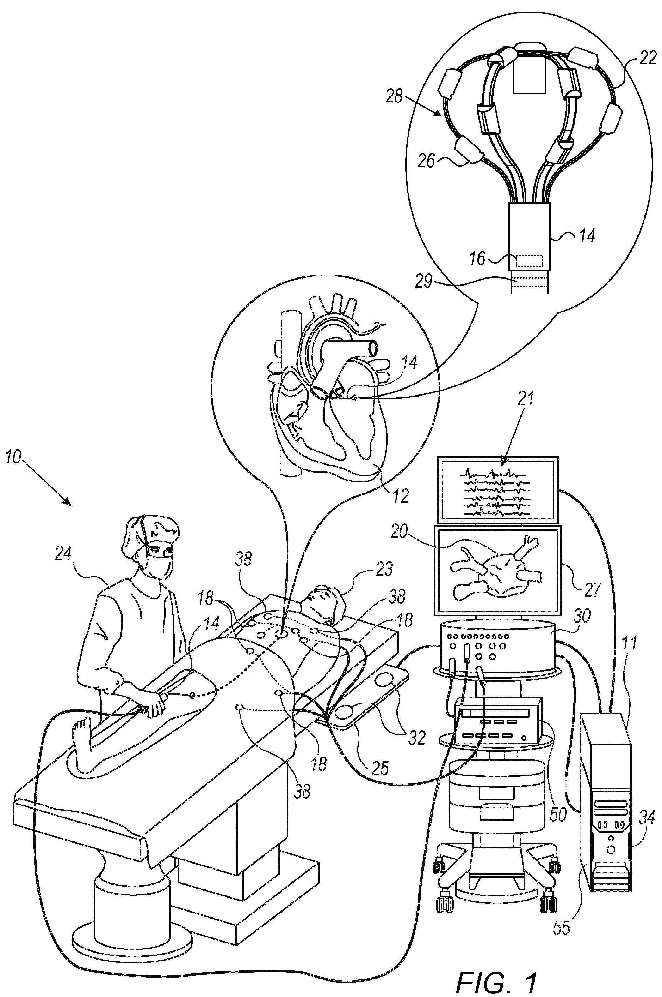

Reference is initially made to Fig. 1, which is a schematic illustration of an example electrophysiology mapping and ablation system 10, in accordance with some examples of the present disclosure. One commercial product embodying elements of system 10 is available as the CARTO^{™} 3 System, available from Biosense Webster, Inc., 31A Technology Drive, Irvine, CA 92618.

System 10 comprises one or more catheters, which are percutaneously inserted by a physician 24 into the body of a subject 23. Following the insertion of each catheter, physician 24 navigates the catheter through the vascular system of subject 23 into a chamber or vascular structure of the subject's heart 12. Typically, the catheters are navigated through a delivery sheath (not shown). The catheters may include a catheter for sensing intracardiac electrogram (IEGM) signals, a catheter for ablating cardiac tissue, and/or a catheter for both sensing and ablating.

For example, an exemplary catheter 14 configured for both sensing and pulsed field ablation (PFA) causing irreversible electroporation (IRE) is shown in Fig. 1. Catheter 14 comprises multiple electrodes 26 optionally distributed over a plurality of splines 22 at a distal end 28 of the catheter. Electrodes 26 are configured to sense IEGM signals from the tissue and to apply PFA pulses to the tissue, e.g., as described in co-assigned US Application 17/559,558 to Govari et al.

Typically, catheter 14 further comprises a tracking sensor 29 for tracking the three-dimensional (3D) location and orientation of distal end 28. Tracking sensor 29 may be embedded in or near distal end 28.

Typically, tracking sensor 29 comprises three magnetic coils. A location pad 25, which comprises a plurality of magnetic coils 32 configured to generate a magnetic field in a predefined working volume, is positioned near (e.g., underneath) subject 23. Based on signals induced in the coils by the magnetic field, the location and orientation of distal end 28 is tracked. Details of such magnetic-based tracking are described in U.S. Patent Nos. 5,5391,199, 5,443,489, 5,558,091, 6,172,499, 6,239,724, 6,332,089, 6,484,118, 6,618,612, 6,690,963, 6,788,967, and 6,892,091.

Typically, system 10 further comprises one or more electrode patches 38 configured to contact the skin of subject 23. Patches 38 may establish a location reference for location pad 25. Additionally, electrical current from electrodes 26 may be sensed at patches 38, and the location of each electrode 26 may be triangulated in response thereto. This location information may be combined with the information derived from the magnetic-based tracking described above so as to increase the precision of the tracking. Details of such impedance-based tracking technology are described in US Patent Nos. 7,536,218, 7,756,576, 7,848,787, 7,869,865, and 8,456,182.

System 10 further comprises a recorder 11 and a display 27. Recorder 11 is configured to record electrocardiographic (ECG) signals 21 acquired by body-surface ECG electrodes 18 and/or IEGM signals acquired by electrodes 26 of catheter 14, and optionally, to display these signals on display 27. Recorder 11 may also be configured to pace heart 12 and/or may be electrically connected to a standalone pacer.

System 10 further comprises an ablation-energy generator 50 configured to conduct ablative energy to one or more electrodes at the distal end of an ablation catheter, such as electrodes 26. Energy produced by ablation-energy generator 50 may include, but is not limited to, radiofrequency (RF) energy and/or PFA energy, including monopolar or bipolar high-voltage direct-current (DC) pulses for effecting IRE, or combinations thereof. Ablation-energy generator 50 comprises energy-generating circuitry configured to produce the energy, along with a controller configured to control the circuitry and, optionally, perform other computational functions.

System 10 further comprises a workstation 55 comprising a processor 34, a volatile memory and/or non-volatile memory that may store appropriate software instructions, and a user interface. Processor 34 may be configured to perform multiple functions, including, for example, (1) mapping the endocardial anatomy of heart 12 in 3D and rendering the resulting anatomical map 20 for display on display 27, (2) displaying, on display 27, activation sequences and/or other data compiled from ECG signals 21 in representative visual indicia or imagery superimposed on anatomical map 20, (3) displaying the real-time location and orientation of one or more catheters within the body of subject 23, and (4) displaying sites of interest, such as sites at which ablation energy has been applied.

System 10 further comprises a patient interface unit (PIU) 30, which is configured to establish electrical communication between power supplies, workstation 55, and the electrophysiological equipment belonging to the system. The electrophysiological equipment may comprise, for example, one or more catheters, location pad 25, ECG electrodes 18, electrode patches 38, ablation-energy generator 50, and/or recorder 11. Typically, PIU 30 further comprises another processor configured to compute of location and orientation of each of the catheters and to perform any relevant computations based on ECG signals 21.

In general, the term "processor," as used in the description and claims below, may refer to a single processor, such as processor 34, the controller of ablation-energy generator 50, or the processor of PIU 30. Alternatively, this term may refer to a cooperatively networked or clustered set of processors. For example, any of the functionality described hereinbelow may be performed cooperatively by some or all of processor 34, the controller of ablation-energy generator 50, and the processor of PIU 30.

The functionality of each of the processors described herein may be implemented solely in hardware, e.g., using one or more fixed-function or general-purpose integrated circuits, Application-Specific Integrated Circuits (ASICs), and/or Field-Programmable Gate Arrays (FPGAs). Alternatively, this functionality may be implemented at least partly in software. For example, the processor may be embodied as a programmed processor comprising, for example, a central processing unit (CPU) and/or a Graphics Processing Unit (GPU). Program code, including software programs, and/or data may be loaded for execution and processing by the CPU and/or GPU. The program code and/or data may be downloaded to the processor in electronic form, over a network, for example. Alternatively or additionally, the program code and/or data may be provided and/or stored on non-transitory tangible media, such as magnetic, optical, or electronic memory. Such program code and/or data, when provided to the processor, produce a machine or special-purpose computer, configured to perform the tasks described herein.

### APPLYING PFA

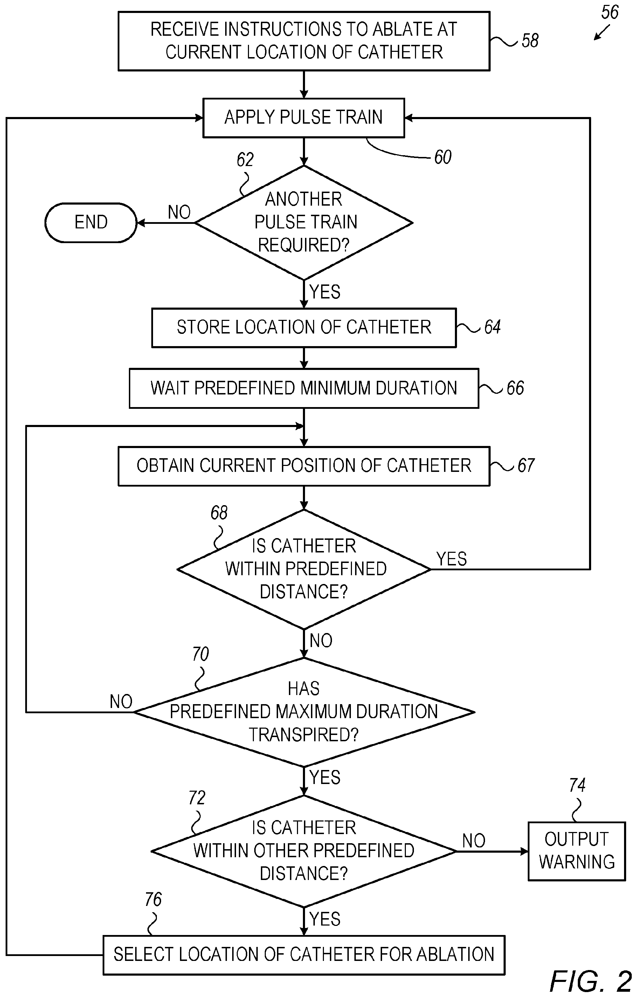

Reference is now made to Fig. 2, which is a flow diagram for a method 56, executed by a processor, for applying PFA to tissue of heart 12 using catheter 14 or any other suitable catheter, in accordance with some examples of the present disclosure.

Typically, method 56 begins with an instruction-receiving step 58, at which the processor receives instructions, from a user, to ablate tissue at the current location of the catheter. For example, subsequently to navigating the catheter to the desired location in the heart, physician 24 (Fig. 1) may enter the instructions, e.g., by pressing a foot pedal, pressing a button on the handle of the catheter, or touching a touch screen belonging to display 27. In response to the instructions, the processor begins to apply PFA to the tissue.

The processor is configured to apply PFA by applying a series of PFA pulse trains. In the context of the present application, including the claims, a "pulse train" refers to a series of one or more pulses.

The processor applies each pulse train, using the catheter, at a train-applying step 60. Subsequently to applying the pulse train, the processor checks, at a checking step 62, whether another pulse train is required, typically by comparing the number of delivered pulse trains to a predefined number. If not, method 56 ends. Otherwise, the processor stores the location of the catheter at a location-storing step 64, and then waits a predefined minimum duration, at a waiting step 66, so as not to apply the next pulse train too soon after the most recent pulse train.

Following waiting step 66, the processor tracks the position of the catheter. In other words, the processor repeatedly (i) obtains (i.e., receives or computes) the current position of the catheter at a position-obtaining step 67, and (ii) checks, at another checking step 68, whether this position is within a predefined distance from the location at which a previous pulse train was applied. (Further details regarding checking step 68 are provided below.) Thus, based on the tracking, the processor may detect that the catheter is within the predefined distance following the predefined minimum duration from the most recent pulse train. In response thereto, the processor may return to train-applying step 60.

If, on the other hand, the processor ascertains, based on the tracking, that the catheter is not within the predefined distance, the processor checks, at another checking step 70, whether a predefined maximum duration has transpired. If not, the processor returns to checking step 68. Otherwise, the processor may output a warning and/or select another location for application of PFA.

For example, the processor may check, at another checking step 72, whether the catheter is within another (larger) predefined distance from the previous location. If not, the processor may output a warning at an outputting step 74, e.g., by displaying the warning on display 27 (Fig. 1). Otherwise, the processor may, at a selecting step 76, select the location of the catheter for application of another series of PFA pulse trains. The processor may then return to train-applying step 60, optionally after receiving, from the user, approval to ablate at the new location.

Alternatively, in response to ascertaining, at checking step 70, that the predefined maximum duration has transpired, the processor may perform outputting step 74 without performing checking step 72.

In some examples, at checking step 68, the processor checks whether the catheter is within the predefined distance from the location at which the first pulse train was applied. (In such examples, location-storing step 64 may be performed only for the first pulse train of the series.) In other examples, the processor checks whether the catheter is within the predefined distance from the location at which the most recent pulse train was applied. In yet other examples, the processor checks whether the catheter is within the predefined distance from both locations, such that the next pulse train is applied only if the catheter is within the predefined distance from both locations. In yet other examples, the processor checks whether the catheter is within the predefined distance from either one of the locations, such that the next pulse train is applied provided the catheter is within either one of the locations.

In general, the predefined minimum duration, maximum duration, and distance depend on the amplitude and duration of each pulse train. In some examples, the predefined minimum duration is between 0.5 and 2 s, the predefined maximum duration is between 1 and 3 s, and/or the predefined distance is between 1 and 4 mm.

As described above, per method 56, the processor applies a subsequent pulse train upon the catheter being within the predefined distance, regardless of whether the catheter is continuing to move toward the previous location.

In other examples, the processor applies the subsequent pulse train in response to the probe reaching a minimum distance from the previous location. In other words, even after the catheter is within the predefined distance, the processor refrains from applying the next pulse train provided the catheter is continuing to move toward the previous location (and provided that the maximum duration has not yet been reached).

In some examples, processor tracks the force applied to the catheter by the tissue. For example, the processor may continually receive a force-indicating signal from a pressure transducer 16 at the distal end of the catheter. In such examples, for each of the pulse trains, the processor may apply the pulse train in response to the force exceeding a predefined lower threshold and/or being less than a predefined higher threshold. For example, prior to executing train-applying step 60, the processor may check whether the force exceeds the lower threshold and/or is less than the higher threshold. If yes, the processor may apply the pulse train; otherwise, the processor may refrain from applying the pulse train

It will be appreciated by persons skilled in the art that the present disclosure is not limited to what has been particularly shown and described hereinabove. Rather, the scope of the present disclosure includes both combinations and subcombinations of the various features described hereinabove, as well as variations and modifications thereof that are not in the prior art, which would occur to persons skilled in the art upon reading the foregoing description

## Claims

1. A system (10) for applying a series of pulse field ablation (PFA) pulse trains to tissue of a heart (12), the system comprising:
energy-generating circuitry (50); and
a processor (30, 34, 50), configured to:
using the energy-generating circuitry and a catheter (14) in the heart, apply one of the pulse trains,
store a location of the catheter at which the one of the pulse trains is applied,
track a position of the catheter following the application of the one of the pulse trains,
based on the tracking, detect that the catheter is within a predefined distance from the location following a predefined minimum duration from a most recent one of the pulse trains, and
in response to the detecting, apply another one of the pulse trains using the energy-generating circuitry and the catheter.

2. The system (10) according to claim 1, wherein the processor (30, 34, 50) is configured to detect that the catheter (14) is within the predefined distance prior to a predefined maximum duration from the most recent one of the pulse trains.

3. The system (10) according to claim 2, wherein the predefined maximum duration is between 1 and 3 s.

4. The system (10) according to any of claims 1 to 3, wherein the predefined distance is between 1 and 4 mm.

5. The system (10) according to any of claims 1 to 4, wherein the predefined minimum duration is between 0.5 and 2 s.

6. The system (10) according to any of claims 1 to 5, wherein the processor (30, 34, 50) is configured to apply the other one of the pulse trains in response to the catheter (14) reaching a minimum distance from the location.

7. The system (10) according to any of claims 1 to 6,
wherein the series is a first series and the location is a first location, and
wherein the processor (30, 34, 50) is further configured to:
apply a pulse train of a second series of PFA pulse trains using the energy-generating circuitry (50) and the catheter (14) in the heart (12),
store a second location of the catheter at which the pulse train of the second series is applied,
track the position of the catheter following the application of the pulse train of the second series,
based on the tracking, ascertain that the catheter is not within the predefined distance from the second location following the predefined minimum duration, and prior to a predefined maximum duration, from a most recent pulse train of the second series, and
in response to the ascertaining, output a warning.

8. The system (10) according to any of claims 1 to 6,
wherein the series is a first series and the location is a first location, and
wherein the processor (30, 34, 50) is further configured to:
apply a pulse train of a second series of PFA pulse trains using the catheter (14) in the heart (12),
store a second location of the catheter at which the pulse train of the second series is applied,
track the position of the catheter following the application of the pulse train of the second series,
based on the tracking, ascertain that the catheter is not within the predefined distance from the second location following the predefined minimum duration, and prior to a predefined maximum duration, from a most recent pulse train of the second series, and
in response to the ascertaining, and in response to the catheter being at a third location within another predefined distance from the second location, select the third location for application of a third series of PFA pulse trains.

9. The system (10) according to any of claims 1 to 8, wherein the processor (30, 34, 50) is further configured to track a force applied to the catheter (14) by the tissue, and wherein the processor is further configured to apply each of the pulse trains in response to the force exceeding a predefined threshold.

## Patentansprüche

1. System (10) zum Anwenden einer Serie von Pulsfeldablationsimpulsfolgen (PFA-Impulsfolgen) auf Gewebe eines Herzens (12), wobei das System umfasst:
Energieerzeugungsschaltlogik (50); und
einen Prozessor (30, 34, 50), der konfiguriert ist zum:
Anwenden einer der Impulsfolgen unter Verwendung der Energieerzeugungsschaltlogik und eines Katheters (14) in dem Herzen,
Speichern einer Position des Katheters, an der die eine der Impulsfolgen angewendet wird,
Nachverfolgen einer Position des Katheters nach der Anwendung der einen der Impulsfolgen,
basierend auf der Nachverfolgung, Erkennen, dass sich der Katheter innerhalb einer vordefinierten Entfernung von der Position befindet, die einer vordefinierten Mindestdauer seit der letzten der Impulsfolgen folgt, und
als Reaktion auf das Erkennen, Anwenden einer weiteren der Impulsfolgen unter Verwendung der Energieerzeugungsschaltlogik und des Katheters.

2. System (10) nach Anspruch 1, wobei der Prozessor (30, 34, 50) konfiguriert ist, um zu erkennen, dass sich der Katheter (14) innerhalb der vordefinierten Entfernung vor einer vordefinierten maximalen Dauer seit der letzten der Impulsfolgen befindet.

3. System (10) nach Anspruch 2, wobei die vordefinierte maximale Dauer zwischen 1 und 3 s liegt.

4. System (10) nach einem der Ansprüche 1 bis 3, wobei die vordefinierte Entfernung zwischen 1 und 4 mm beträgt.

5. System (10) nach einem der Ansprüche 1 bis 4, wobei die vordefinierte Mindestdauer zwischen 0,5 und 2 s beträgt.

6. System (10) nach einem der Ansprüche 1 bis 5, wobei der Prozessor (30, 34, 50) konfiguriert ist, um die andere der Impulsfolgen als Reaktion darauf anzuwenden, dass der Katheter (14) eine Mindestentfernung von der Position erreicht.

7. System (10) nach einem der Ansprüche 1 bis 6,
wobei die Serie eine erste Serie und die Position eine erste Position ist, und
wobei der Prozessor (30, 34, 50) ferner konfiguriert zum:
Anwenden einer Impulsfolge einer zweiten Serie von PFA-Impulsfolgen unter Verwendung der Energieerzeugungsschaltlogik (50) und des Katheters (14) in dem Herzen (12),
Speichern einer zweiten Position des Katheters, an der die Impulsfolge der zweiten Serie angewendet wird,
Nachverfolgen der Position des Katheters nach der Anwendung der Impulsfolge der zweiten Serie,
basierend auf der Nachverfolgung, Feststellen, dass sich der Katheter nach der vordefinierten Mindestdauer und vor einer vordefinierten Höchstdauer aus einer letzten Impulsfolge der zweiten Serie nicht innerhalb der vordefinierten Entfernung von der zweiten Position befindet, und
als Reaktion auf die Feststellung, Ausgeben einer Warnung.

8. System (10) nach einem der Ansprüche 1 bis 6,
wobei die Serie eine erste Serie und die Position eine erste Position ist, und
wobei der Prozessor (30, 34, 50) ferner konfiguriert zum:
Anwenden einer Impulsfolge einer zweiten Serie von PFA-Impulsfolgen unter Verwendung des Katheters (14) in dem Herzen (12),
Speichern einer zweiten Position des Katheters, an der die Impulsfolge der zweiten Serie angewendet wird,
Nachverfolgen der Position des Katheters nach der Anwendung der Impulsfolge der zweiten Serie,
basierend auf der Nachverfolgung, Feststellen, dass sich der Katheter nach der vordefinierten Mindestdauer und vor einer vordefinierten Höchstdauer aus einer letzten Impulsfolge der zweiten Serie nicht innerhalb der vordefinierten Entfernung von der zweiten Position befindet, und
als Reaktion auf die Feststellung und als Reaktion darauf, dass sich der Katheter an einer dritten Position innerhalb einer weiteren vordefinierten Entfernung von der zweiten Position befindet, Auswählen der dritten Position für die Anwendung einer dritten Serie von PFA-Impulsfolgen.

9. System (10) nach einem der Ansprüche 1 bis 8, wobei der Prozessor (30, 34, 50) ferner konfiguriert ist, um eine durch das Gewebe auf den Katheter (14) ausgeübte Kraft nachzuverfolgen, und wobei der Prozessor ferner konfiguriert ist, um jede der Impulsfolgen als Reaktion auf die Kraft anzuwenden, die einen vordefinierten Schwellenwert überschreitet.

## Revendications

1. Système (10) permettant d'appliquer une série de trains d'impulsions d'ablation par champ pulsé (PFA) à un tissu d'un cœur (12), le système comprenant :
un système de circuits de génération d'énergie (50) ; et
un processeur (30, 34, 50), configuré pour :
à l'aide du système de circuits de génération d'énergie et d'un cathéter (14) dans le cœur, appliquer l'un des trains d'impulsions,
stocker une localisation du cathéter au niveau duquel celui précité des trains d'impulsions est appliqué,
suivre une position du cathéter à la suite de l'application de celui précité des trains d'impulsions,
en fonction du suivi, détecter que le cathéter se trouve à moins d'une distance prédéfinie de la localisation suivant une durée minimale prédéfinie à partir d'un plus récent parmi les trains d'impulsions, et
en réponse à la détection, appliquer un autre des trains d'impulsions à l'aide du système de circuits de génération d'énergie et du cathéter.

2. Système (10) selon la revendication 1, dans lequel le processeur (30, 34, 50) est configuré pour détecter que le cathéter (14) se trouve à moins de la distance prédéfinie avant une durée maximale prédéfinie à partir du plus récent des trains d'impulsions.

3. Système (10) selon la revendication 2, dans lequel la durée maximale prédéfinie est comprise entre 1 et 3 s.

4. Système (10) selon l'une quelconque des revendications 1 à 3, dans lequel la distance prédéfinie est comprise entre 1 et 4 mm.

5. Système (10) selon l'une quelconque des revendications 1 à 4, dans lequel la durée minimale prédéfinie est comprise entre 0,5 et 2 s.

6. Système (10) selon l'une quelconque des revendications 1 à 5, dans lequel le processeur (30, 34, 50) est configuré pour appliquer l'autre des trains d'impulsions en réponse au cathéter (14) atteignant une distance minimale par rapport à la localisation.

7. Système (10) selon l'une quelconque des revendications 1 à 6,
dans lequel la série est une première série et la localisation est une première localisation, et
dans lequel le processeur (30, 34, 50) est configuré en outre pour :
appliquer un train d'impulsions d'une deuxième série de trains d'impulsions de PFA à l'aide du système de circuits de génération d'énergie (50) et du cathéter (14) dans le cœur (12),
stocker une deuxième localisation du cathéter au niveau de laquelle le train d'impulsions de la deuxième série est appliqué,
suivre la position du cathéter à la suite de l'application du train d'impulsions de la deuxième série,
en fonction du suivi, déterminer que le cathéter ne se trouve pas à moins de la distance prédéfinie de la deuxième localisation suivant la durée minimale prédéfinie, et avant une durée maximale prédéfinie, à partir d'un train d'impulsions le plus récent de la deuxième série, et
en réponse à la détermination, délivrer en sortie un avertissement.

8. Système (10) selon l'une quelconque des revendications 1 à 6,
dans lequel la série est une première série et la localisation est une première localisation, et
dans lequel le processeur (30, 34, 50) est configuré en outre pour :
appliquer un train d'impulsions d'une deuxième série de trains d'impulsions de PFA à l'aide du cathéter (14) dans le cœur (12),
stocker une deuxième localisation du cathéter au niveau de laquelle le train d'impulsions de la deuxième série est appliqué,
suivre la position du cathéter à la suite de l'application du train d'impulsions de la deuxième série,
en fonction du suivi, déterminer que le cathéter ne se trouve pas à moins de la distance prédéfinie de la deuxième localisation suivant la durée minimale prédéfinie, et avant une durée maximale prédéfinie, à partir d'un train d'impulsions le plus récent de la deuxième série, et
en réponse à la détermination, et en réponse au cathéter étant au niveau d'une troisième localisation à moins d'une autre distance prédéfinie de la deuxième localisation, sélectionner la troisième localisation pour application d'une troisième série de trains d'impulsions de PFA.

9. Système (10) selon l'une quelconque des revendications 1 à 8, dans lequel le processeur (30, 34, 50) est configuré en outre pour suivre une force appliquée au cathéter (14) par le tissu, et dans lequel le processeur est configuré en outre pour appliquer chacun des trains d'impulsions en réponse à la force dépassant un seuil prédéfini.
